# EUROPEAN PATENT SPECIFICATION

(11) **EP 1 614 439 B1**
(45) Date of publication and mention of the grant of the patent: **06.08.2008**
(21) Application number: 05253648.9
(22) Date of filing: 14.06.2005
(51) Int. Cl.: A61M 5/32

(54) **Needle removal device**
Nadelentfernungsvorrichtung
Dispositif pour extraction des aiguilles

(30) Priority: 19.06.2004 GB 0413759
(43) Date of publication of application: 11.01.2006
(73) Proprietor: Cooley, Stephen Francis Gerald, Vantage Business Park Banbury Oxfordshire OX16 9UX (GB); Field, Philip William, Vantage Business Park Banbury Oxfordshire OX16 9UX (GB)
(72) Inventor: Cooley, Stephen Francis Gerald, Vantage Business Park Banbury Oxfordshire OX16 9UX (GB); Field, Philip William, Vantage Business Park Banbury Oxfordshire OX16 9UX (GB)
(74) Representative: Stanley, Michael Gordon

(56) References cited:
- EP-A- 0 441 628
- EP-A- 1 321 157
- BE-A3- 1 007 352
- DE-C1- 19 733 241
- US-A- 4 809 915
- US-A- 5 482 207
- US-A1- 2005 121 343

## Description

### Field

This invention provides a means of safely removing used needles from the body of a multi-use syringe and single use syringe without the need to undo and remove the needle manually

### Background of the Invention

Multi-use and single use syringes are used in medicine, veterinary practice and in dentistry. In dentistry multi-use syringes are used for the administration of anaesthetic injections into the mouth, or may be used for mass-vaccination programs. Devices are known for the removal of used disposable needles from the syringe. Typically a needle disposal container may be provided with a lid having a shaped aperture for engagement with a needle hub. The technician or nurse carefully inserts the needle into the aperture so that the needle hub is held stationary within the aperture and then unscrews the syringe from the needle hub and the needle drops into the disposal container.

In another known apparatus shown in FR 2609401, there is disclosed a disposal container fitted with a needle gripping device that facilitates needle removal. EP-A-0838 228 discloses a motorised needle removal device which uses serrated arms for engaging the needle hub for unscrewing the needle. The needles then fall freely into the container after removal. However, if the frictional engagement is insufficient to unscrew the needle hub then the needle will remain in place. An alternative unscrewing action is shown in US 5,531,323 in which a rotatable gear wheel engages the external surface of the needle hub. Another device is shown in US 5482 207A which has three gripping arms operated by cams. A needle removal apparatus according to the preamble of claim 1 is shown in BE-A3-1007 352.

The applicants earlier patent application GB-A-2376 892 describes apparatus for the removal of a needle assembly having a needle hub which is screw-threadedly engaged with a syringe, said apparatus comprising a housing having an opening for insertion of the needle assembly and adjacent portion of the syringe, a socket mounted within the body in alignment with the opening, said socket having a cavity internally shaped to receive the hub and a coaxial through-bore to accommodate the needle, the socket holding the hub rotationally fast relative thereto allowing the syringe to be unscrewed from the needle assembly, and an ejector means located in the housing for ejecting the unscrewed needle assembly from the socket into a collection container located within the body.

The present invention provides an automatic needle removal device in which the needle can be removed from the syringe body by rotation of the needle hub and can be collected in a collection bin and which is a development of the earlier apparatus described above.

### Statements of Invention

According to the present invention there is provided apparatus for the removal from a syringe of a needle assembly having a needle hub attached to the body of the syringe, according to claim 1.

The term syringe covers both syringes with disposable plastic bodies typically having push on needle hubs and multi-use re-usable syringes, typically made of metal and/or glass and having screw on needle hubs.

Preferably the socket comprises two semi-circular jaws which are rotated by cam means which close the jaws in one direction of rotation relative to the socket to remove the needle assembly and which open the jaws in the reverse direction of rotation. The two jaws may also be biased apart by spring means.

Preferably the jaws have an internal profile shaped to accommodate a plurality of different sized hubs.

The jaw housing may include cam surfaces which act on the jaws during said limited rotational movement to open and close the jaws.

The motor is preferably a suitable electrical bi-directional motor which rotates the housing through a gear train clamp the hub and unscrew the hub from the needle body in one direction and release the hub in the other direction. The motor is preferably a 12V motor torque sensing motor which may be operated from the mains via a transformer or may be battery operated, preferably using rechargeable batteries.

Preferably, the jaw housing has a gear formed integrally therewith.

A second manual switch may be located externally of the apparatus housing.

The apparatus housing may in use sit directly on the collection bin, the bin having an aperture for receiving the needle assemblies, and which may be provided with a rotatable closure which also serves as said second switch for the motor, the motor being activated when the closure is in an open condition. The motor may be operable only when both the switches are closed.

The housing may be connected to the collection bin by a locking means which is unlocked to allow the bin to be removed from the housing for disposal of needle assemblies. Additionally or alternatively, the housing may be connected a resilient clip means on one of the housing and bin which engages a detent on the other of the housing and bin.

The locking means may also be provided by the closure which when open couples the housing to the bin and closed releases the housing from the collection bin for detachment therefrom.

The apparatus housing preferably includes a motor support plate which is shaped to locate the motor, the shaped portion of the support plate being received in a like recess formed in the collection bin to facilitate location of the apparatus on the location bin.

The collection bin may be provided as a part of said apparatus according to the present invention.

The bin may be made from a suitable plastic material to enable both bin and contents to be incinerated, and for withstanding elevated temperatures used for cleaning

### Description of Drawings

The invention will be described by way of example and with reference to the accompanying drawings in which:
- Fig. 1: is an isometric view of apparatus according to the present invention also showing the insertion of a syringe and needle,
- Fig. 2: is an exploded isometric view of the apparatus of Fig.1 ,
- Fig. 3: is a plan view of the apparatus of Fig.1 with the housing removed to show the drive mechanism for the needle receiving socket,
- Fig. 4: is a section on the line IV-IV of Fig.3
- Fig. 5: is a section on the line V-V of Fig. 3
- Fig. 6: is a side elevation of the apparatus with the housing removed to show the internal workings, and
- Fig.7: is an exploded isometric view showing the needle removal assembly in an assembled condition.

### Detailed Description of the Invention

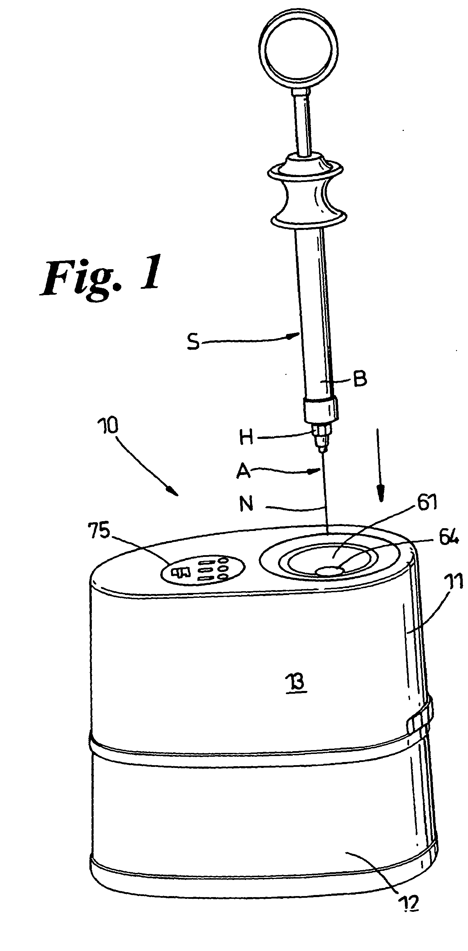

With reference to Fig. 1, there is shown a typical dental syringe S having a needle assembly A screwed onto the body B of the syringe. The needle assembly comprises needle N which is mounted within a hub H which screws onto the body B. Disposable needle assemblies A are removable from the body by gripping or holding the external surface of the hub H and unscrewing the hub.

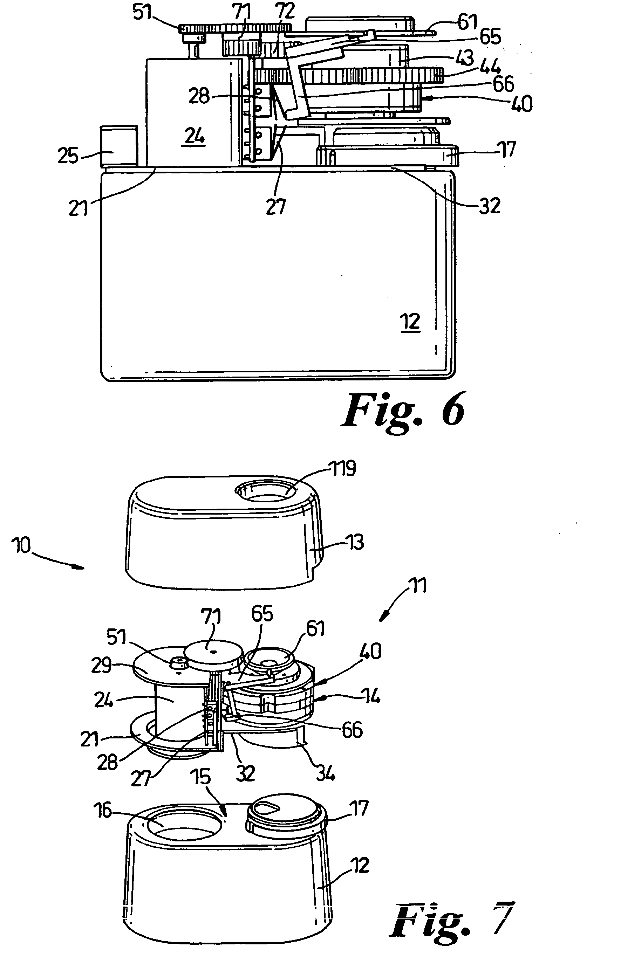

With reference also to Fig 7, the needle assembly A is removed from the re-usable syringe S using the needle remover apparatus 10. The syringe S may be used for medical, dental or veterinary purposes. The apparatus 10 is formed in two distinct parts, a needle remover 11 and a collection bin 12 on which the needle remover 11 sits. The needle remover 11 comprises a hollow housing 13 and a subassembly 14.

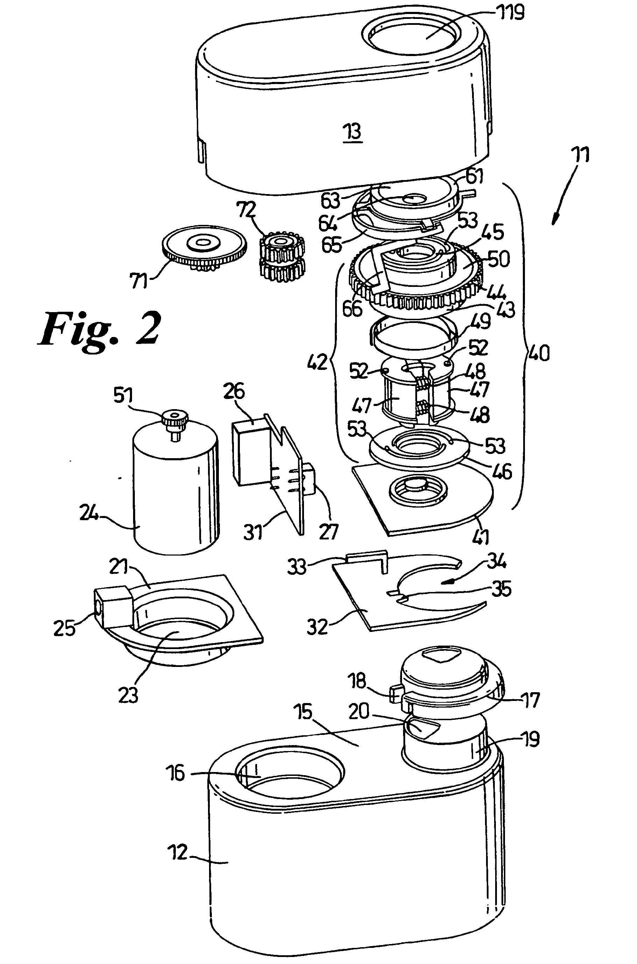

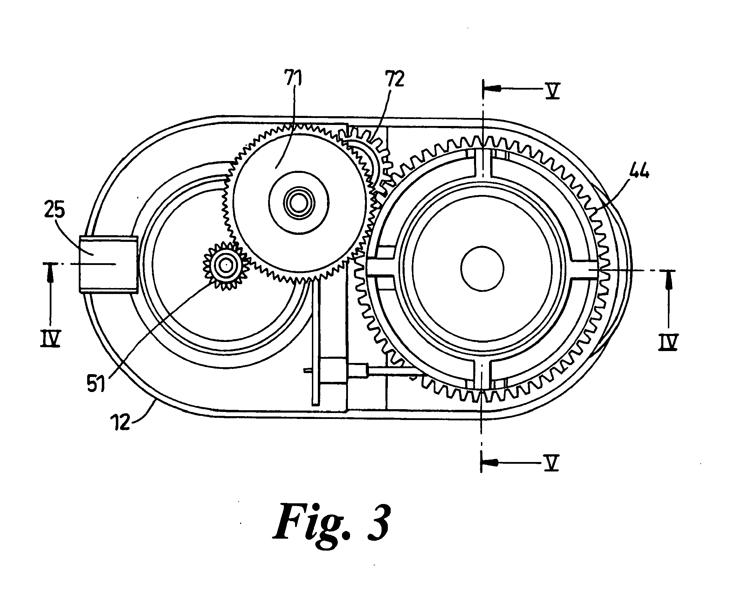

Referring now to Figs 2-6 also, the sub assembly 14 comprises a base plate 21 which has a recess 23 therein and supports an upright PCB (printed circuit board) 31 on its inner side. The recess 23 in use locates in a like recess 16 in the top surface 15 of the bin 12 and houses an electric motor 24 in an upright condition. Also located on the base plate 21 is a electrical power socket 25 for a power connection to the PCB 31. The PCB 31 includes an AC/DC transformer 26, electrical contacts 27,28, and programmable microprocessor (not shown) for control of the motor 24. The motor 24 is held in its upright position and has a shaft with a drive gear 51 attached thereto.

A switch plate 32 sits on the upper surface 15 of the bin 12 to the side of the base plate 21 and includes an arcuate clip portion 34 which in use clips around a raised circular boss 19 on the upper surface 15 of the bin 12. The boss 19 has an aperture 20 therein and has a closure 17 mounted on the boss for opening and closing the aperture 20. The switch plate 32 includes a leg 33 which is engagable with a contact 27 for operation of the motor 24 and a tab 35 which in use engages a recess 18 in the outer surface of the closure 17 such that rotation of the closure 17 on the boss 19 causes the lug 33 to open or close the contact 27.

A gripping jaw assembly 40 for gripping the needle hub H is mounted above the closure 17. The assembly 40 includes a base plate 41 on which a housing 42 for the jaws is rotatably mounted. The base plate 41 in use clips into the housing 42. The jaw housing 42 comprises a cylindrical hollow body 43 having a coaxial outer flange 50 with a gear 44 on its radially outer surface, and annular cam plates 45,46 at its upper and lower ends. A pair of arcuate jaws 47 are located within coaxially within the cylindrical housing 42. The two jaws 47 are biased apart by four springs 48 and may additionally also be biased apart by a spiral spring 49. The upper and lower ends of the two jaws 47 are provided with lugs or pins 52 which engage cam surfaces provided in slots 53 (see Fig.2) in the respective cam plates 45 46. Rotation of the housing 42 relative to the jaws 47 causes the lugs 52 to move along the slots 53 simultaneously moving the jaws 47 radially relative to the axis of rotation of the housing 42.

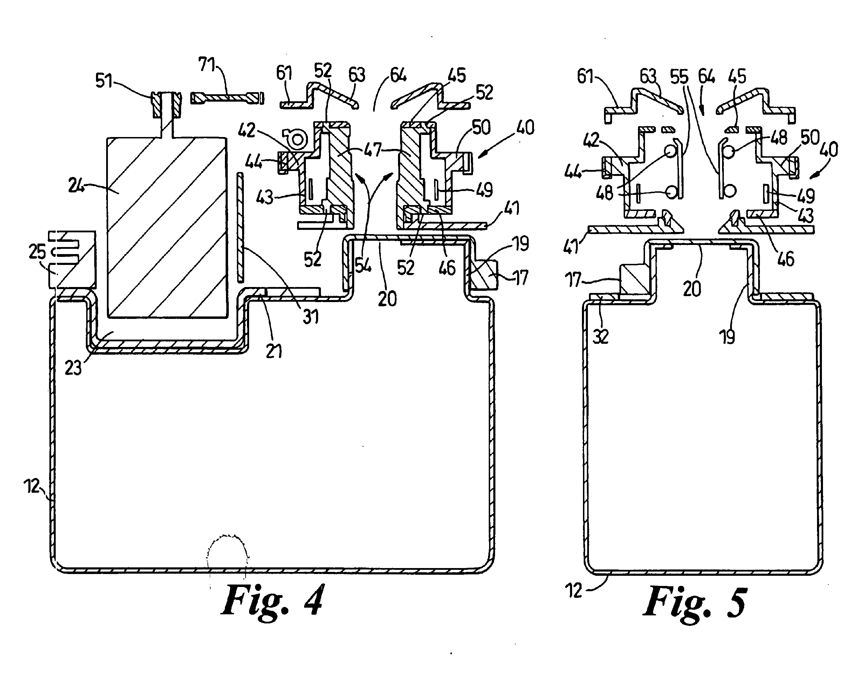

The two jaws 47 each have a guide plate 55 thereon to assist and guide movement of the jaws 47 towards and way from each other. The jaws 47 form a generally cylindrical socket located within the housing 13 in alignment with an opening 119 in the top surface of the housing 13. The two jaws 47 form a split open ended socket having a internal stepped profile 54 (see Fig.4) which can accept a plurality of different sized needle hubs H. The stepped profile 54 forms a split cavity with the larger diameter portion open to the opening 119 and the smallest diameter portion at the base of the cavity.

An annular activation plate 61 is slidably mounted on the flange 50 on the housing 42 by means of pins (not shown). The plate 61 is capable of limited axial movement relative to the housing 42 and is biased away from the flange 50 by springs arranged coaxially of the pins. The movement away from the flange 50 may be limited by abutments on the pins. The activation plate 61 fills the opening 119 in the housing 13 and has a downwardly extending conical portion 63 with an aperture 64 in the centre which aligns with the cavity between the jaws 47 and the aperture 119 in the housing 13. A semi-circular switch means 65 is pivotally mounted to the activation plate 61 and has a dog leg shaped limb 66 thereon which in use activates contact 28 on the PCB 31 causing the motor 24 to operate.

The housing 42 is caused to rotate by operation of the motor 24. The motor 24 is preferably a bi-directional motor which is torque sensitive delivering a suitable maximum torque load removal of a needle N. The operation of the motor is controlled by the programmed controller on the PCB 31. The gear wheel 51 on the motor is connected to the gear 44 on the housing 42 by gears 71, 72, the gear 71 being a reduction gear.

The PCB 31 may be connected to an LED display 75 on the top of the housing 13 which indicates various stages of operation of the needle remover apparatus 10.

In use, the apparatus 10 is first activated by rotation of the closure switch 17 which opens the aperture 20 and causes the leg 33 to close contact 27 on the PCB. This may illuminate a warning LED on the display 75.

A needle N is inserted through the opening 119 in the housing 13 and aperture 64 until the needle body B engages the activation plate 61. Further inward movement of the needle body B depresses the activation plate 61 locating the needle hub H between the jaws 47 and causing the switch means 65 to move the limb 66 to operate contact 28 on the PCB. The needle N passes through the gap between the jaws 47.

The closure of contact 28 operates the motor 24 which via gears 51,71,72 rotates the jaw housing 42 in one direction. The jaws 47 are connected to the housing 42 by the lugs 52 engaging in the slots 53 in the cam plates 45,46. Limited rotation of the housing 42 relative to the jaws 47 moves the lugs along the slots 53 moving the jaws together to clamp the hub H. Once the lugs 52 abut the end of the respective slot 53, further rotation of the housing 42 unscrews the needle and hub from the syringe body. The motor 24 is programmed to rotate the housing through an appropriate number of rotations over about a 3sec. time period.

After the programmed rotation in one direction, the microprocessor operates a warning LED on the display 75 and the syringe S is then removed. The motor 24 operates in the reverse direction opening the jaws 47. On opening of the jaws, the needle N will eject from the jaws and drop into the collection bin 12.
The bin 12 can be removed from the apparatus when full. The closure 17 closes the aperture 20 and the bin and its contents can be disposed of by any suitable method e.g. incineration.

The assembly 40 comprising the jaw housing 42, activation plate 61 and switch means 65 can be removed from the housing 13 as a unit for cleaning.

The apparatus may also be used with disposable syringes having needles whose needle hub is a push fit onto the syringe body. Rotation of the hub "unscrews" the neddle assembly from the syringe body.

## Claims

1. Apparatus(10) for the removal from a syringe (S) of a needle assembly (A) having a needle hub(H) attached to the body (B) of the syringe (S), said apparatus (10) comprising a motor (24), a housing (13) having an opening (119) for insertion of the needle assembly (A) and adjacent portion of the syringe (S), a needle receiving socket (47) formed from at least two jaws (47) mounted within the housing (13) in alignment with the opening (119), said socket having a cavity internally shaped to receive the needle hub (H) and accommodate the needle (N) to hold the hub (H) rotationally fast relative thereto, said jaws(47) being located in a housing (42) which is rotatable by the motor (24) and configured for being moved together to grip the needle hub to facilitate unscrewing of the needle hub to remove the needle assembly from the syringe, and for being moved apart to eject the unscrewed needle assembly into a collection bin (12), the motor (24) being activatable by manual switch means (61) (45) located inside of the opening (119) in the housing (13) and configured to be operated by insertion of the syringe body into said opening (119), **characterised in that** the housing (42) for the jaws, the jaws (47) and switch means (61,45) are removable as a unit from the apparatus housing for cleaning.

2. Apparatus as claimed in Claim 1 wherein the socket comprises two semi-circular jaws (47) which are rotatable by cam means (52,53) which close the jaws (47) in one direction of rotation and which open the jaws (47) in the other direction of rotation.

3. Apparatus as claimed in Claim 1 or Claim 2 wherein the two jaws (47) are also biased apart by spring means (48, 49) .

4. Apparatus as claimed in any one of Claims 1 to 3 wherein the semi-circular jaws (47) have an internal profile (54) enabling the jaws to be utilised with a plurality of different needle hubs (H).

5. Apparatus as claimed in any one of Claims 1 to 4 wherein the housing for the jaws is capable of limited rotational movement relative to the jaws (47) and includes cam surfaces (53) which act on the jaws (47) during said limited rotational movement to open and close the jaws.

6. Apparatus as claimed in Claim 5 wherein the motor (24) is an electrical bi-directional motor which is configured to rotate the housing (42) via a gear train (44,71,72).

7. Apparatus as Claimed in Claim 5 or Claim 6 wherein the housing (42) has a gear (44) formed integrally therewith on its outer surface.

8. Apparatus as claimed in any one of Claims 1 to 7, wherein the switch means comprises an activation plate (61) which is movable axially on insertion of the syringe S to activate a contact (28) to operate the motor.

9. Apparatus as claimed in any of Claims 1 to 8, wherein the apparatus housing (13) sits directly on the collection bin (12), the bin (12.) having an aperture (20) for receiving the needle assemblies(A) and which is provided with a rotatable closure (17) which also serves as a second switch means for the motor (24), the motor (24) being activatable when the bin closure (17) is in an open condition.

10. Apparatus as claimed in Claim 9, wherein the motor (24) is operable only when both the switch means (17,61,45) are closed.

11. Apparatus as claimed in Claim 9 or Claim 10, wherein the apparatus housing (13) includes a motor support plate (21) which is shaped to locate the motor (24), the shaped portion of the support plate (21) being received in a like recess (23) formed in the collection bin (12) to facilitate location of the apparatus (10) on the collection bin (12).

12. Apparatus as claimed in any one of Claims 1 to 10 further including the collection bin (12).

## Patentansprüche

1. Vorrichtung (10) zum Entfernen einer Nadelbaugruppe (A), die eine am Körper (B) der Spritze (S) angebrachte Nadelnabe (H) aufweist, aus einer Spritze (S), wobei die Vorrichtung (10) aufweist: einen Motor (24); ein Gehäuse (13) mit einer Öffnung (119) für das Einsetzen der Nadelbaugruppe (A) und des benachbarten Abschnittes der Spritze (S); eine Nadelaufnahmebuchse (47), die aus mindestens zwei Klemmbacken (47) gebildet wird, die innerhalb des Gehäuse (13) in Ausrichtung mit der Öffnung (119) montiert sind, wobei die Buchse einen Hohlraum aufweist, der innen geformt ist, um die Nadelnabe (H) aufzunehmen, und um die Nadel (N) aufzunehmen, um die Nabe (H) rotationsbeständig relativ dazu zu halten, wobei die Klemmbacken (47) in einem Gehäuse (42) angeordnet sind, das mittels des Motors (24) gedreht werden kann, und so ausgebildet sind, dass sie zusammen bewegt werden, um die Nadelnabe zu ergreifen, um das Abschrauben der Nadelnabe zu erleichtern, um die Nadelbaugruppe von der Spritze zu entfernen, und dass sie auseinander bewegt werden, um die abgeschraubte Nadelbaugruppe in einen Sammelbehälter (12) zu werfen, wobei der Motor (24) mittels eines manuellen Schaltmittels (61) (45) aktiviert werden kann, das innerhalb der Öffnung (119) im Gehäuse (13) angeordnet und so ausgebildet ist, dass es durch Einsetzen des Spritzenkörpers in die Öffnung (119) betätigt wird, **dadurch gekennzeichnet, dass** das Gehäuse (42) für die Klemmbacken, die Klemmbacken (47) und das Schaltmittel (61, 45) als eine Einheit aus dem Vorrichtungsgehäuse für eine Reinigung entfernt werden können.

2. Vorrichtung nach Anspruch 1, bei der die Buchse zwei halbkreisförmige Klemmbacken (47) aufweist, die mittels eines Nockenmittels (52, 53) gedreht werden können, das die Klemmbacken (47) in einer Rotationsrichtung schließt und die Klemmbacken (47) in der anderen Rotationsrichtung öffnet.

3. Vorrichtung nach Anspruch 1 oder Anspruch 2, bei der die zwei Klemmbacken (47) ebenfalls mittels eines Federmittels (48, 49) auseinandergelenkt werden.

4. Vorrichtung nach einem der Ansprüche 1 bis 3, bei der die halbkreisförmigen Klemmbacken (47) ein Innenprofil (54) aufweisen, das in die Lage versetzt, dass die Klemmbacken mit einer Vielzahl von unterschiedlichen Nadelnaben (H) genutzt werden können.

5. Vorrichtung nach einem der Ansprüche 1 bis 4, bei der das Gehäuse für die Klemmbacken zu einer begrenzten Rotationsbewegung relativ zu den Klemmbacken (47) in der Lage ist und Nockenflächen (53) umfasst, die auf die Klemmbacken (47) während der begrenzten Rotationsbewegung wirken, um die Klemmbacken zu öffnen und zu schließen.

6. Vorrichtung nach Anspruch 5, bei der der Motor (24) ein elektrischer Umkehrmotor ist, der ausgebildet ist, um das Gehäuse (42) mittels eines Getriebezuges (44, 71, 72) zu drehen.

7. Vorrichtung nach Anspruch 5 oder Anspruch 6, bei der das Gehäuse (42) ein Zahnrad (44) aufweist, das zusammenhängend damit auf seiner äußeren Fläche gebildet wird.

8. Vorrichtung nach einem der Ansprüche 1 bis 7, bei der das Schaltmittel eine Aktivierungsplatte (61) aufweist, die axial beim Einsetzen der Spritze (S) beweglich ist, um einen Kontakt (28) zu aktivieren, um den Motor zu betätigen.

9. Vorrichtung nach einem der Ansprüche 1 bis 8, bei der das Vorrichtungsgehäuse (13) direkt auf dem Sammelbehälter (12) sitzt, wobei der Behälter (12) eine Öffnung (20) für das Aufnehmen der Nadelbaugruppen (A) aufweist, und der mit einem drehbaren Verschluss (17) versehen ist, der ebenfalls als ein zweites Schaltmittel für den Motor (24) dient, wobei der Motor (24) aktiviert werden kann, wenn der Behälterverschluss (17) in einem offenen Zustand ist.

10. Vorrichtung nach Anspruch 9, bei der der Motor (24) erst betätigt werden kann, wenn beide Schaltmittel (17, 61, 45) geschlossen sind.

11. Vorrichtung nach Anspruch 9 oder Anspruch 10, bei der das Vorrichtungsgehäuse (13) eine Motorhalteplatte (21) umfasst, die geformt ist, um den Motor (24) anzuordnen, wobei der geformte Abschnitt der Halteplatte (21) in einer gleichen Aussparung (23) aufgenommen wird, die im Sammelbehälter (12) gebildet wird, um die Anordnung der Vorrichtung (10) auf dem Sammelbehälter (12) zu erleichtern.

12. Vorrichtung nach einem der Ansprüche 1 bis 10, die außerdem den Sammelbehälter (12) umfasst.

## Revendications

1. Dispositif (10) pour extraire d'une seringue (S) un ensemble d'aiguille (A) comprenant un moyeu d'aiguille (H) fixé sur le corps (B) de la seringue (S), ledit dispositif (10) comprenant un moteur (24), un boîtier (13) pourvu d'une ouverture (119) pour l'insertion de l'ensemble d'aiguille (A) et d'une partie adjacente de la seringue (S), une douille de réception d'aiguille (47) formée par au moins deux mâchoires (47) montées à l'intérieur du boîtier (13) en alignement avec l'ouverture (119), ladite douille ayant une cavité intérieurement profilée pour recevoir le moyeu d'aiguille (H) et recevoir l'aiguille (N) de manière à maintenir le moyeu (H) fixe en rotation par rapport à celle-ci, lesdites mâchoires (47) étant situées dans un logement (42) que le moteur (24) peut faire tourner et configurées pour être déplacées ensemble pour saisir le moyeu d'aiguille pour faciliter le dévissage du moyeu d'aiguille pour extraire l'ensemble d'aiguille de la seringue et pour être écartées pour éjecter l'ensemble d'aiguille dévissé dans une boîte de collecte (12), le moteur (24) pouvant être activé par un moyen de commutation manuel (61) (45) situé à l'intérieur de l'ouverture (119) dans le boîtier (13) et configuré pour fonctionner sous l'effet de l'insertion du corps de seringue dans ladite ouverture (119), **caractérisé en ce que** le logement (42) pour les mâchoires, les mâchoires (47) et le moyen de commutation (61, 45) peuvent être retirés en tant qu'unité du boîtier de dispositif en vue d'un nettoyage.

2. Dispositif selon la revendication 1, dans lequel la douille comprend deux mâchoires semi-circulaires (47) qui peuvent effectuer une rotation sous l'action d'un moyen de came (52, 53) qui ferme les mâchoires (47) dans une direction de rotation et ouvre les mâchoires (47) dans l'autre direction de rotation.

3. Dispositif selon la revendication 1 ou la revendication 2, dans lequel les deux mâchoires (47) sont également contraintes de s'écarter par un moyen élastique (48, 49).

4. Dispositif selon l'une quelconque des revendications 1 à 3, dans lequel les mâchoires semi-circulaires (47) ont un profil intérieur (54) qui permet aux mâchoires d'être utilisées avec une pluralité de moyeux d'aiguille (H) différents.

5. Dispositif selon l'une quelconque des revendications 1 à 4, dans lequel le logement pour les mâchoires est capable d'un mouvement de rotation limité par rapport aux mâchoires (47) et inclut des surfaces de came (53) qui agissent sur les mâchoires (47) pendant ledit mouvement de rotation limité pour ouvrir et fermer les mâchoires.

6. Dispositif selon la revendication 5, dans lequel le moteur (24) est un moteur bidirectionnel électrique qui est configuré pour faire tourner le logement (42) par le biais d'un train d'engrenages (44, 71, 72).

7. Dispositif selon la revendication 5 ou la revendication 6, dans lequel un engrenage (44) est formé de manière intégrale avec le logement (42) sur sa surface extérieure.

8. Dispositif selon l'une quelconque des revendications 1 à 7, dans lequel le moyen de commutation comprend une plaque d'activation (61) qui peut être déplacée axialement lors de l'insertion de la seringue (S) pour activer un contact (28) pour actionner le moteur.

9. Dispositif selon l'une quelconque des revendications 1 à 8, dans lequel le boîtier de dispositif (13) est posé directement sur la boîte de collecte (12), la boîte (12) ayant une ouverture (20) pour recevoir les ensembles d'aiguille (A) et étant pourvue d'une fermeture rotative (17) servant également de second moyen de commutation pour le moteur (24), le moteur (24) pouvant être activé lorsque la fermeture de boîte (17) est dans une condition ouverte.

10. Dispositif selon la revendication 9, dans lequel le moteur (24) peut fonctionner uniquement lorsque les deux moyens de commutation (17, 61, 45) sont fermés.

11. Dispositif selon la revendication 9 ou la revendication 10, dans lequel le boîtier de dispositif (13) inclut une plaque de support de moteur (21) qui est profilée pour positionner le moteur (24), la partie profilée de la plaque de support (21) étant reçue dans un creux identique (23) formé dans la boîte de collecte (12) pour faciliter le positionnement du dispositif (10) sur la boîte de collecte (12).

12. Dispositif selon l'une quelconque des revendications 1 à 10, incluant, en outre, la boîte de collecte (12).
